# EUROPEAN PATENT SPECIFICATION

(11) **EP 3 915 578 B1**
(45) Date of publication and mention of the grant of the patent: **02.12.2026**
(21) Application number: 21176238.0
(22) Date of filing: 27.05.2021
(51) Int. Cl.: C07K 14/705, A61K 40/11, A61K 40/31, A61K 40/42, A61P 35/00, A61P 37/06

(54) **CHIMERIC ANTIGEN RECEPTOR WITH A SPACER COMPRISING C2-SET IG-LIKE DOMAINS**
CHIMÄRER ANTIGENREZEPTOR MIT EINEM ABSTANDSHALTER MIT C2-SET-IG-ÄHNLICHEN DOMÄNEN
RÉCEPTEUR D'ANTIGÈNE CHIMÉRIQUE DOTÉ D'UN ESPACEUR COMPRENANT DES DOMAINES D'ENSEMBLE C2 DE TYPE IG

(30) Priority: 28.05.2020 EP 20177214
(43) Date of publication of application: 01.12.2021
(73) Proprietor: Miltenyi Biotec B.V. & Co. KG, 51429 Bergisch Gladbach (DE)
(72) Inventor: PFEIFER, Rita, 51429 Bergisch Gladbach (DE); SCHÄFER, Daniel, 51429 Bergisch Gladbach (DE); JOHNSTON, Ian, 51429 Bergisch Gladbach (DE)
(74) Representative: Biervert, Christian

(56) References cited:
- WO-A1-2017/064084
- PATEL S D ET AL: "IMPACT OF CHIMERIC IMMUNE RECEPTOR EXTRACELLULAR PROTEIN DOMAINS ON T CELL FUNCTION", GENE THERAPY, NATURE PUBLISHING GROUP, LONDON, GB, vol. 6, no. 6, 1 March 1999 (1999-03-01), pages 412 - 419, XP001095026, ISSN: 0969-7128, DOI: 10.1038/SJ.GT.3300831
- SONIA GUEDAN ET AL: "Engineering and Design of Chimeric Antigen Receptors", MOLECULAR THERAPY - METHODS & CLINICAL DEVELOP, vol. 12, 15 March 2019 (2019-03-15), GB, pages 145 - 156, XP055605656, ISSN: 2329-0501, DOI: 10.1016/j.omtm.2018.12.009
- GRAZYNA LIPOWSKA-BHALLA ET AL: "Targeted immunotherapy of cancer with CAR T cells: achievements and challenges", CANCER IMMUNOLOGY, IMMUNOTHERAPY, SPRINGER, BERLIN, DE, vol. 61, no. 7, 22 April 2012 (2012-04-22), pages 953 - 962, XP035074376, ISSN: 1432-0851, DOI: 10.1007/S00262-012-1254-0
- LÄUBLI HEINZ ET AL: "Sialic acid-binding immunoglobulin-like lectins (Siglecs) detect self-associated molecular patterns to regulate immune responses", CMLS CELLULAR AND MOLECULAR LIFE SCIENCES, BIRKHAUSER VERLAG, HEIDELBERG, DE, vol. 77, no. 4, 4 September 2019 (2019-09-04), pages 593 - 605, XP037038988, ISSN: 1420-682X, [retrieved on 20190904], DOI: 10.1007/S00018-019-03288-X
- DANIEL SCHÄFER ET AL: "A Novel Siglec-4 Derived Spacer Improves the Functionality of CAR T Cells Against Membrane-Proximal Epitopes", FRONTIERS IN IMMUNOLOGY, vol. 11, 7 August 2020 (2020-08-07), CH, XP055727823, ISSN: 1664-3224, DOI: 10.3389/fimmu.2020.01704

## Description

### Field of the invention

The present invention relates to the field of chimeric antigen receptors (CARs) having a spacer between antigen binding domain and transmembrane domain, in particular having a spacer that comprises at least one C2-set Ig-like domain.

### Background of the invention

While CAR therapies have now achieved public recognition, their development and the quest for optimal CAR design has been a multistep process. Ever since their initial description in 1989 by Eshhar and colleagues (Gross et al., 1989), the receptors have evolved from a two-chimeric-TCR chain architecture to a one-protein design which commonly incorporates a single-chain variable fragment (scFv) of a given antibody as the antigen binding moiety, an extracellular spacer and a transmembrane region as structural features, as well as signal transduction units for T cell activation. Originally, the spacer domain was introduced into the CAR framework as an inert building block to allow the antigen binding moiety to extend beyond the T cell's glycocalyx and improve antigen accessibility. Following this assumption, a plethora of spacer regions was designed simultaneously ranging from the immunoglobulin (Ig) domains of the crystallizable fragments (Fc) of antibodies to extracellular domains of CD8α, CD28, the TCRβ chain or NKG2D and were applied without comparative analyses. However, already very early on, Patel and colleagues provided the scientific proof that the spacer region can be of paramount importance for the receptor function and affects its expression, surface stability, and signal transduction (Patel et al., 1999). Patel et al (1999) analyzed chimeric immune receptors (CIRs), wherein the heterologous protein spacer domains were selected from IgG1, CD4, CD7 and CD8. More recent accumulating research has further been showing that in addition to the nature of the spacer, effective antigen recognition depends on the functional interplay between the spatial localization of the target epitope and the CAR spacer length.

The use of Ig-derived spacers is particularly attractive as it provides the opportunity to modulate the spacer length into long (CH2-CH3 domain), medium (CH3) and short (hinge only) structures, while retaining the nature of the parent protein. However, Ig-derived spacers have faced various complications during their development. In particular, off-target activation, CAR T cell sequestration in the lung, tonic signaling and activation-induced cell death (AICD) have been described leading to only a limited T cell persistence (Hudecek et al., 2015; Watanabe et al., 2016). Although these effects could be abrogated by mutating the amino acid sequence essential for FcR binding, it needs to be taken into consideration that these experiments were conducted in immunosuppressed NSG mice and whether FcR binding can be completely eliminated in humans remains unclear.

WO2017064084A1 discloses a spacer for a CAR comprising domains 2, 3 and/or 4 of CD4. A CD4 spacer contains C2-set IgG-like domains and V-like domains.

Guedan, S. et al (2019, Molecular Therapy: Methods & Clinical Development, 12: 145-156) is a review article about CARs and discloses that many CARs use IgG-derived spacers consisting of two Ig-like domains, CH2 and CH3, have been used for efficient antigen recognition and are especially useful in assessing the level of CAR expression at the surface of the T cell. Alternatively, CD28 and CD8alpha have been extensively used as spacers.

Lipowska-Bhalla, G. et al (2012, Cancer Immunol Immunother 61:953-962) is another review article about CARs and discloses that spacer regions used tend to be comprised of common Ig-like domains due to the stability of the protein and have included immunoglobulin Fc or an extracellular fragment derived from CD28, TCRbeta chain, CD8alpha, or NKG2D.

Of note, several clinical studies that used IgG-derived spacers described only limited anti-tumor efficacy and low CAR T cell persistence. In fact, the only currently approved CAR T cell-based therapies use CD28 (Yescarta) and CD8 (Kymriah) as spacer domains. In addition, a severe side effect that may occur during CAR T cell therapy with state-of-the-art CARs is the cytokine release syndrome (CRS). CRS can complicate treatment of patients and can be a restricting dosing factor, as it is correlated with the number of injected CAR T cells. In worst case, CRS can have a fatal outcome for the patient.

There is a need in the art for an improved or alternative CAR construct which, when expressed in an immune cell, demonstrates lower off-target activation and/or reduces the chances for CRS.

### Brief description of the invention

Surprisingly it was found that a CAR comprising a spacer that preferentially comprises 1 to 3 C2-set Ig-like domains reduces the off-target activation in vitro and in vivo, when expressed in an immune cell and compared to other state-of-the-art CARs. The invention is set out in the appended set of claims. Said spacer shifted phenotypes of said immune cell to a greater extent into a memory phenotype during in vivo expansion with lower exhaustion marker upregulation and lower cytokine release in vitro, while exhibiting the same or better cytotoxicity as compared to other state-of-the-art CARs. The spacer of the CAR of the invention consists of 1, 2 or 3 C2-set Ig-like domain(s), wherein said C2-set Ig-like domain(s) is/are selected from the C2-set Ig-like domain(s) of Siglec-4. Said spacer does not comprise a C1-set Ig-like domain.

### Brief description of the drawings

FIG. 1: Design of C2-set Ig-like domain containing CARs specific for CD20. (A) Modular structure of the CD20 CAR constructs with the C2-set Ig-like domain spacers and extracellular domain comparison of the CAR constructs. (B) Schematic of CD20 specific C2-set Ig-like domain spacer CARs compared to CD8α spacer CAR.
FIG. 2: In vitro evaluation of novel C2-set Ig-like domain CD20 specific Siglec spacer CAR T cells.
   (A-B) Expression analysis of the CAR constructs in transiently transfected HEK293T cells 24 h post transfection (A) and in transduced T cells (B) from two donors 6 days post transduction. LNGFR and CAR expression were evaluated by flow cytometry. (C-E) C2-set Ig-like domain spacer CAR T cells were cocultured with Raji or HEK293T cells for 5 h at a ratio of 1:1 and T cell expression of CD107a (C) and intracellular TNF-α (D) were analyzed by flow cytometry. (E) The frequency of TIM3, LAG3 and PD1 triple positive CAR T cells was analyzed after 24 h co-culture at a 1:2 ratio of CAR T cells to Raji or HEK293T cells by flow cytometry. CAR T cells alone were also cultured in order to exclude tonic signaling/unspecific activation. n = 3. Error bars, mean ± SD. ns > 0.05, *p < 0.05, **p < 0.01, ***p < 0.001 and ****p < 0.0001 [one-way ANOVA, CAR T + Raji compared to Untreated (UnTd)].

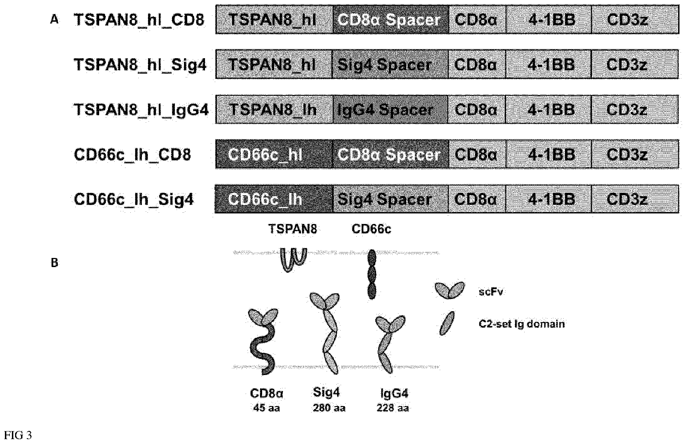

FIG. 3: Design of C2-set Ig-like domain containing CARs specific for CD66c and TSPAN8. (A) Modular structure of the CD66c and TSPAN8 CAR constructs with the C2-set Ig-like domain spacers and extracellular domain comparison of the CAR constructs. (B) Schematic of CD66c and TSPAN8 specific C2-set Ig-like domain spacer CARs compared to CD8α and IgG4 CH2-CH3 spacer CAR.

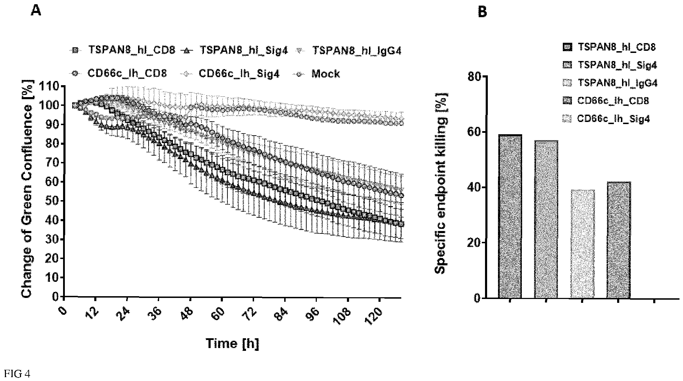

FIG. 4: Dynamic monitoring of in vitro cytotoxicity mediated by T cells expressing different spacer CAR constructs directed against TSPAN8 or CD66c. (A) On day 11 after T cell transduction, AsPC1 target cells stably expressing eGFP were co-cultured with different CAR T cell groups at an effector to target cell ratio of 2:1 and fluorescence emission was measured in the IncuCyte^{®} imaging platform for 132 hours with 2 hours interval. Co-cultures with untransduced T cells served as negative control. Shown is one representative from 2 separate experiments and 6 donors in total. (B) After 132 hours of co-culture (endpoint), the total killing efficacy was determined compared to the control group.

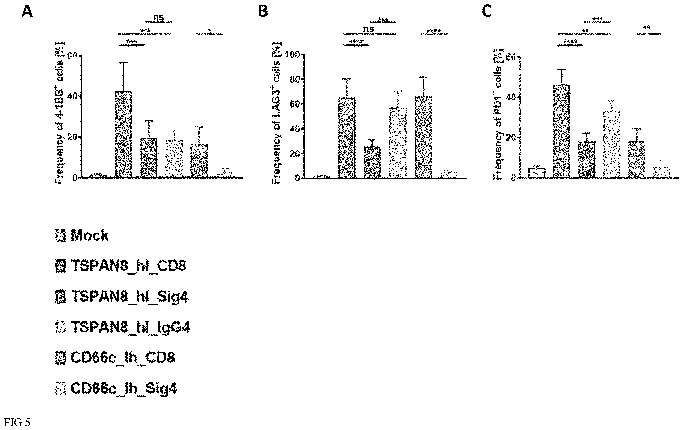

FIG. 5: In vitro comparison of T cells transduced with TSPAN8 and CD66c CAR constructs, incorporating different spacer domains. Frequency of (A) 4-1BB, (B) LAG3 and (C) PD1 positive CAR T cells was analyzed at the end of the cytolytic evaluation with AsPC1 cells by flow cytometry. Shown is the mean ± SD. ns > 0.05, *p < 0.05, **p < 0.01, ***p < 0.001 and ****p < 0.0001 [one-way ANOVA, multiple comparisons].

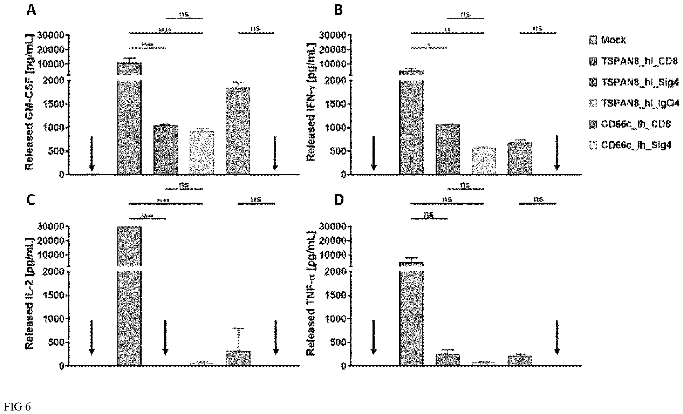

FIG. 6: Cytokine secretion by CAR-transduced T cells following antigen stimulation. On day 11 following transduction, TSPAN8 and CD66c CAR T cells incorporating different spacer domains were co-cultured with the target cell line AsPC1 at an effector to target cell ratio of 1:2 for 24 hours and culture supernatants were analyzed for cytokine release using the MACSPlex technique. Cultures of non-modified T cell with target cells served to assess the specificity of CAR-mediated lymphocyte response. Secretion pattern for (A) GM-CSF, (B) IFNγ, (C) IL-2, and (D) TNFα is shown. Data is representative for n=2. Shown is the mean ± SD. ns > 0.05, *p < 0.05, **p < 0.01, ***p < 0.001 and ****p < 0.0001 [one-way ANOVA, multiple comparisons].

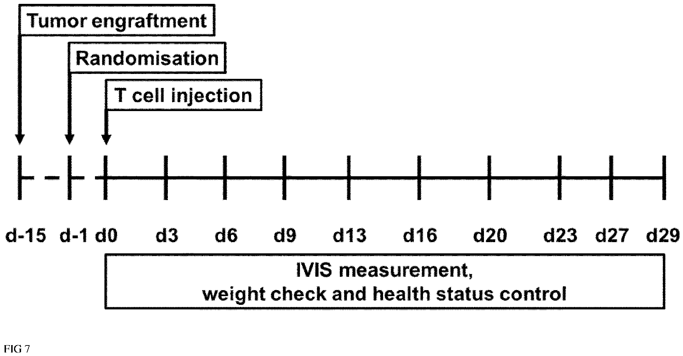

FIG. 7: Outline of the experimental design to study the therapeutic potential of different spacer CAR T cells in a subcutaneous xenograft model.

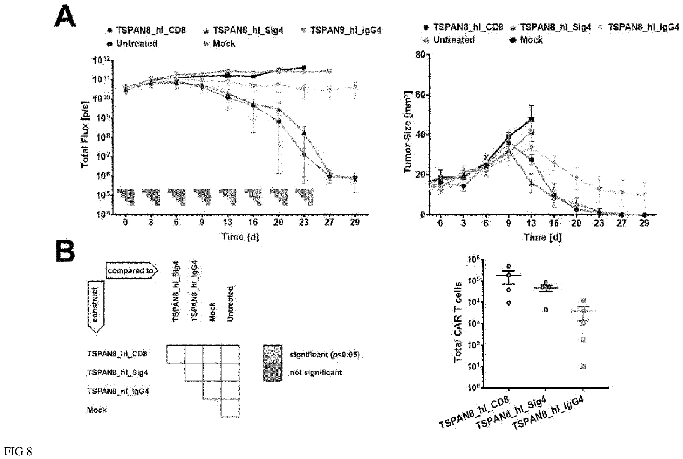

FIG. 8: The TSPAN8 specific Siglec-4 spacer CAR T cells exhibit the same anti-tumor efficacy as the CD8α spacer CAR T cells, while retaining a more memory-like phenotype. (A) Tumor burden and change in tumor size over time after TSPAN8 CAR T cell infusion. Untreated and Mock T cell treated animals served as controls, T cells from one donor were used. IgG4: n = 5; Sig4 and CD8α: n=4. PSM p<0.05 (green) [one-way ANOVA, multiple comparisons]. Organization of the pairwise significant matrix for group comparison of in vivo performance of TSPAN8_hl_CD8, TSPAN8_hl_Sig4, TSPAN8_hl_IgG4, Untreated and Mock. PSM p<0.05 (green) [one-way ANOVA, multiple comparisons]. (B) Total number of CAR positive T cells recovered from spleens of TSPAN8 CAR-treated animals at the end of the experiment calculated after flow cytometric analysis. IgG4: n = 5; Sig4 and CD8α: n = 4.

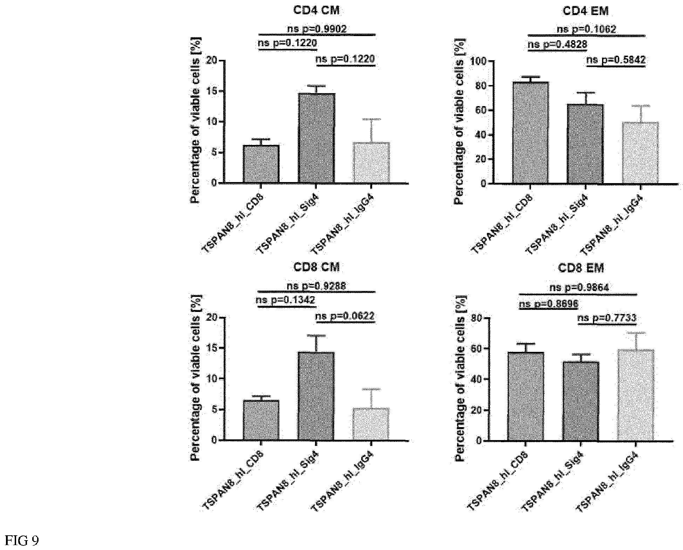

FIG. 9: Phenotypic characterization of TSPAN8-specific CAR T cells incorporating different spacers following in vivo therapeutic treatment. Upon reaching humane endpoint criteria, the spleen of each animal and in each mouse cohort was processed into a single cell suspension and the phenotype of the infiltrating human immune cells was determined. Compared to the CD8 spacer and IgG4 spacer CAR T cell cohort, Siglec-4 spacer CAR-transduced T cells displayed a higher level of central memory cells. n = 4 Shown is the mean ± SD. ns > 0.05, *p < 0.05, **p < 0.01, ***p < 0.001 and ****p < 0.0001 [one-way ANOVA, multiple comparisons].

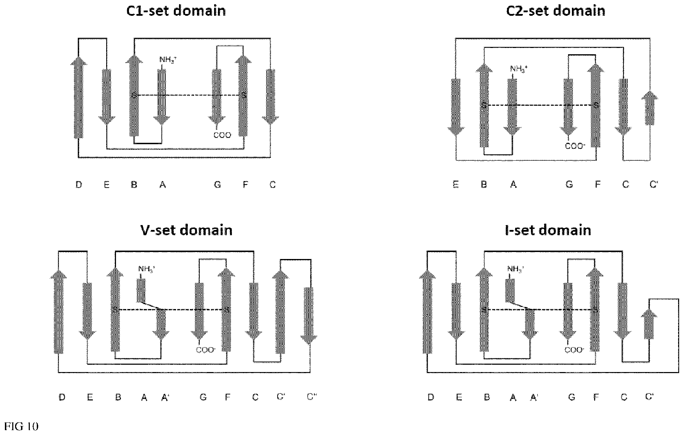

FIG. 10: Ig-like domains are protein domains that are similar in amino acid sequence and structure to the Ig-domains of antibodies. Structurally, the immunoglobulin domain is composed of 7 to 10 β-strands that are arranged as two anti-parallel β-sheets packed tightly against each other forming a β-barrel. The two sheets are linked together by a conserved intradomain disulfide bond. The loops connecting the β-strands display a varying length and character and may form regular reverse turns or short α-helical structures, but in most cases are irregular.
   Ig-like domains are classified according to the number of β-strands into 4 different groups called "sets": C1-set domains are classical Ig-like domains and are composed of β-sheet I constituted by the β-strands A, B, D, and E and β-sheet II formed by the β-strands C, F, and G. Such C1-set domains are found exclusively in molecules involved in the immune system. In C2-set domains, the β-strand D is deleted and replaced by the β-strand C' which is directly connected to β-strand E. In this fold, sheet I is composed of the β-strands A, B, E and sheet II of the β-strands C', C, F and G. The group designated as V-set domain contain the two additional β-strands C' and C" between β-strand C and D and sheet I is formed by the β-strands A, B, E, and D, whereas sheet II is composed of the β-strands C", C', C, F, and G. The I-set domain can be described as a truncated V-set domain which is truncated on sheet II resulting in a missing C" strand. A hallmark of the V- and I-set domain is the A-A' kink in the N-terminal A β-strand.

### Detailed description of the invention

In a first aspect the present invention provides a CAR comprising
a) an antigen binding domain specific for an antigen
b) a spacer
c) a transmembrane domain
d) an intracellular signaling domain,
wherein said spacer consists of 1, 2 or 3 C2-set Ig-like domain(s), wherein said C2-set Ig-like domain(s) is/are selected from the C2-set Ig-like domain(s) of Siglec-4.

The spacer of a CAR may be the peptidic linkage between the extracellular antigen binding domain of a CAR and the transmembrane domain of a CAR.

Said C2-set Ig-like domain is characterized by seven antiparallel beta-strands, presented in two beta-sheets of three (ABE) and four (CC'FG) strands.

Said C1-set Ig-like domain is characterized by eight antiparallel beta-strands, presented in two beta-sheets of four (ABED) and four (CC'FG) strands.

The C1-set Ig-like domain and the C2-set Ig-like domain are also defined in more detail below.

Said antigen binding domain may be antibody or antigen binding fragment thereof such as a svFc or a Fab.

Alternatively said antigen binding domain may be ligand such as a cytokine that can bind to the cognitive receptor present on a target cell or may be an antigen that may be able to bind to an antigen-specific B cell.

Said antigen may be an antigen expressed on the surface of a target cell such as a cancer cell. Said antigen may be a soluble antigen, e.g. a soluble antigen that may be coupled to a solid surface or matrix such as a bead, or a soluble antigen that may allow for cross-linking, i.e. that induces dimerization of the CAR.

Said antigen may be a tagged polypeptide as disclosed herein. Then the antigen binding domain of said CAR may be specific for the tag, and the polypeptide may be bind to a antigen expressed on the surface of a target cell.

Said intracellular (cytoplasmic) signaling domain may comprise at least one primary cytoplasmic signaling domain comprising an immunoreceptor tyrosine-based activation motif (ITAM) and/or at least one co-stimulatory signaling domain.

Said primary cytoplasmic signaling domain of said first CAR may be CD3zeta.

Said at least one co-stimulatory domain of said first CAR, may be selected from the group consisting of ICOS, CD154, CD5, CD2, CD46, HVEM, CD8, CD97, TNFRSF18, CD30, SLAM, DAP10, CD64, CD16, CD89, MyD88, KIR-2DS, KIR-3DS, NKp30, NKp44, NKp46, NKG2D, ICAM, CD27, OX40, 4-1BB, and CD28.

Said intracellular (cytoplasmic) signaling domain may comprise at least one inhibitory endodomain, wherein said at least one inhibitory endodomain is a cytoplasmic signaling domain comprising at least one signal transduction element that inhibits an immune cell or comprising at least one element that induces apoptosis.

In one disclosure, but not part of the invention, the CAR as disclosed herein may comprise a spacer comprising or consisting of 1, 2 or 3 C2-set Ig-like domain(s).

In one disclosure, but not part of the invention, the CAR as disclosed herein may comprise a spacer comprising 1, 2 or 3 C2-set Ig-like domain(s) and no C1-set Ig-like domain.

In one disclosure, but not part of the invention, the CAR as disclosed herein may be a CAR, wherein said antigen binding domain and said spacer are from different proteins.

In one disclosure, but not part of the invention, the CAR as disclosed herein may be a CAR, wherein said C2-set Ig-like domain(s) is/are selected from the group of C2-set Ig-like domains of the sialic acid binding Ig-like lectin (Siglec) family.

In one disclosure, but not part of the invention, the CAR as disclosed herein may be a CAR, wherein said Siglec family may comprise Siglec-1, Siglec-2, Siglec-3, Siglec-4, Siglec-5, Siglec-6, Siglec-7, Siglec-8, Siglec-9, Siglec-10, Siglec-11, Siglec-12, Siglec-13, Siglec-14, Siglec-15, Siglec-16 and Siglec-17.

In an embodiment of the invention the CAR as disclosed herein is a CAR, wherein said C2-set Ig-like domain(s) is/are selected from the C2-set Ig-like domain(s) of Siglec-4, and wherein said spacer consists of 1, 2 or 3 C2-set Ig-like domain(s).

In one embodiment of the invention the CAR as disclosed herein may be a CAR, wherein said spacer may comprise or may consist of SEQ ID NO:1.

In one embodiment of the invention the CAR as disclosed herein may be a CAR, wherein said antigen binding domain may be specific for CD20.

In one embodiment of the invention the CAR as disclosed herein may be a CAR, wherein said antigen binding domain may be specific for CD20, wherein said antigen binding domain may comprise the SEQ ID NO:5.

In one disclosure, but not part of the invention, the CAR as disclosed herein may be a CAR comprising SEQ ID NO:6 or SEQ ID NO:7 or SEQ ID NO:8 or SEQ ID NO:9.

In one embodiment of the invention the CAR as disclosed herein may be a CAR, wherein said antigen binding domain may be specific for TSPAN8.

In one embodiment of the invention the CAR as disclosed herein may be a CAR, wherein said antigen binding domain may be specific for TSPAN8, wherein said antigen binding domain may comprise the SEQ ID NO:10.

In one embodiment of the invention the CAR as disclosed herein may be a CAR comprising SEQ ID NO:11.

T cell activation requires interactions between a TCR and an antigen presenting molecule such as MHC1, presenting an antigen-specific peptide. The binding of these two molecules occurs at a specialized cell-cell junction called an immunological synapse (or immune synapse) which is the interface between an antigen-presenting cell or target cell and a lymphocyte such as a T cell or Natural Killer cell. This synapse comprises at least 2 domains: a central cluster of engaged antigen-specific receptors surrounded by a second domain populated with adhesion molecules.

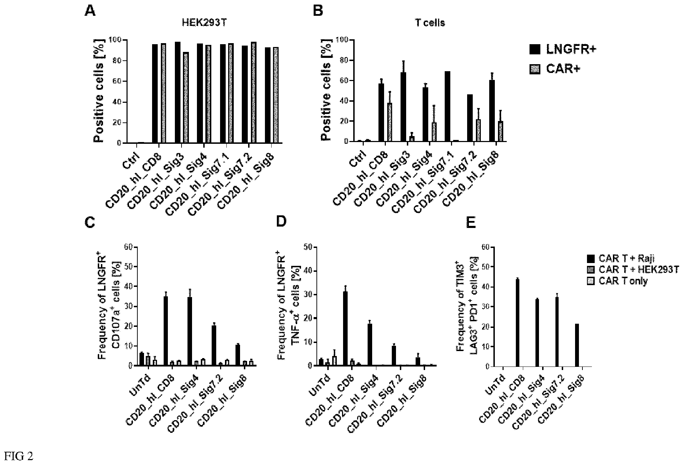

When TCR and MHC1 are engaged, the spacing at their point of interaction is 15nm (Garcia et al, 1996). Microscopical analysis of T cells interacting with antigen presenting cells shows a dynamic structure with nodes of tighter interaction being interspersed with areas where the membrane spacing approaches 100nm (Krummel and Cahalan 2010). However, the parts with larger spacing will allow other molecules to interact (e.g. adhesion molecules, glycocalyx spacing) and not the TCR with the peptide-MHC complex which is strictly limited to about 15nm. But there is evidence that the immunological synapse tolerates a certain flexibility with regard to the spacing of a CAR - antigen interaction leading to a functional spacing also when the distance is about 20 nm or even larger. In Hudecek et al (2013) it is shown that both a CAR comprising a hinge alone and a CAR comprising a CH2 spacer which differ in length by approximately 3.5 nm can kill target cells in vitro. Herein it is also shown in Figure 2C-E that the CD20 CAR is also functional with a spacer comprising one C2-set Ig-like domain or three C2-set Ig-like domains (CD20_hl_Sig7.2 vs. CD20_hl_Sig4) which differ by approximately 7 nm in length.

Said CAR, wherein said antigen binding domain is specific for an epitope on an antigen expressed on a cell membrane of a target cell,
Wherein the total distance of the complex of spacer - antigen binding domain that has bound thereto said antigen via said epitope to said cell membrane of said target cell is according to the formula:
   20 nm ≥ S + A1 + A2 ≥ 7 nm, preferentially 17 nm ≥ S + A1 + A2 ≥ 7 nm, more preferentially 15 nm ≥ S + A1 + A2 ≥ 7 nm,
wherein S is length of spacer in nm, wherein one C2-set domain is about 3.5 nm in size,
Wherein A1 is the length in nm of antigen binding domain and wherein A2 is the distance in nm of the epitope of said antigen from the cell membrane of said target cell.

The total distance of the complex of spacer - antigen binding domain that has bound thereto said antigen via said epitope to said cell membrane of said target cell is equal to the distance between an immune cell and a target cell required to induce functional changes in said immune cell (to form an immunological synapse).

Normally, the length of an antigen binding domain is about 2 nm (between 2 to 4 nm) for a scFv and about 7 nm for a Fab and diabody.

The following embodiments exemplarily demonstrate the use of the formula.

In one embodiment said CAR may be a CAR specific for CD20 comprising three C2-set Ig-like domains, wherein the antigen binding domain is a scFv, wherein A1 = 2nm, wherein A2 = 2.5nm, wherein S=10.5nm.

As the antigen CD20 is a proximal antigen it may be calculated that the distance in nm of the epitope of said antigen from the cell membrane of said target cell may be about 2.5 nm.

In one embodiment said CAR may be a CAR specific for CD20 comprising two C2-set Ig-like domains, wherein the antigen binding domain is a Fab, wherein A1 = 7nm, wherein A2 = 2.5nm, wherein S=7nm.

Said CAR, wherein said antigen binding domain is specific for an epitope on an antigen expressed on a cell membrane of a target cell, wherein said epitope is a relatively proximal epitope, and wherein said spacer comprise two or three C2-set Ig-like domain, and wherein said spacer does not comprise a C1-set Ig-like domain.

Said CAR, wherein said antigen binding domain is specific for an epitope on an antigen expressed on a cell membrane of a target cell, wherein said epitope is a relatively distal epitope, and wherein said spacer comprise one or two C2-set Ig-like domain, and wherein said spacer does not comprise a C1-set Ig-like domain.

Said CAR, wherein said antigen binding domain is specific for an epitope on an antigen expressed on a cell membrane of a target cell, wherein said spacer comprises 1, 2 or 3 C2-set Ig-like domain(s) in dependence of the relatively distal or relatively proximal position of said epitope, and wherein said spacer does not comprise a C1-set Ig-like domain.

The terms "relatively distal" and "relatively proximal" may be used to indicate that there is a significant difference in the relative distance of the two epitopes, when directly compared, from the target cell membrane, significant enough to affect the optimal length of the spacers of the CAR as disclosed herein for optimal functionality of the CAR. The length of the spacer may dependent on the relative position of said epitope, the more distal the epitope the shorter is the spacer of the CAR, the more proximal the epitope the longer is the spacer of the CAR. As described above, the skilled person is aware of the spatial situation of an immunological synapse and would select the suited length of the spacer in dependence of the position of the epitope on the antigen expressed on a cell membrane of a target cell without inventive skills.

Said CAR, wherein the length of the spacer is such that the distance between the cell membranes of the target cell that expresses said antigen and the engineered immune cell that expresses said CAR creates an immune synapse, wherein said spacer does not comprise a C1-set Ig-like domain.

In another aspect the present disclosure, but not part of the invention, provides a nucleic acid encoding a CAR comprising
a) an antigen binding domain specific for an antigen
b) a spacer
c) a transmembrane domain
d) an intracellular signaling domain,
wherein said spacer comprises at least one C2-set Ig-like domain.

In another aspect, the present disclosure, but not part of the invention, provides an immune cell comprising a CAR comprising
a) an antigen binding domain specific for an antigen
b) a spacer
c) a transmembrane domain
d) an intracellular signaling domain,
wherein said spacer comprises at least one C2-set Ig-like domain.

Said immune cell may be a T cell or a NK cell.

In another aspect the present disclosure, but not part of the invention, provides an immune cell comprising a nucleic acid encoding a CAR comprising
a) an antigen binding domain specific for an antigen
b) a spacer
c) a transmembrane domain
d) an intracellular signaling domain,
wherein said spacer comprises at least one C2-set Ig-like domain.

In another aspect the present disclosure, but not part of the invention, provides a pharmaceutical composition comprising immune cells expressing a CAR comprising
a) an antigen binding domain specific for an antigen
b) a spacer
c) a transmembrane domain
d) an intracellular signaling domain,

wherein said spacer comprises at least one C2-set Ig-like domain,
and optionally a pharmaceutical acceptable carrier.

In one aspect the present disclosure, but not part of the invention, provides a method for treating a subject suffering from a disease triggered by a target cell, the method comprising administering to said subject an immune cell expressing a CAR comprising
a) an antigen binding domain specific for an antigen expressed on the surface of said target cell
b) a spacer
c) a transmembrane domain
d) an intracellular signaling domain,
wherein said spacer comprises at least one C2-set Ig-like domain.

Said disease may be cancer and said target cell may be a cancer cell.

In one disclosure, but not part of the invention, the immune cells expressing the CAR as disclosed herein are for use in treatment of a disease associated with a target cell of a subject suffering from said disease, the disease may be e.g. cancer and the target cell a cancerous cell. Immune cells, e.g. T cells or NK cells of a subject may be isolated. The subject may e.g. suffer from said cancer or may be a healthy subject. These cells are genetically modified *in vitro* or *in vivo* to express the CAR as disclosed herein. These engineered cells may be activated and expanded *in vitro* or *in vivo.* In a cellular therapy these engineered cells are infused to a recipient in need thereof. These cells may be a pharmaceutical composition (said cell plus pharmaceutical acceptable carrier). The infused cells may be e.g. able to kill (or at least stop growth of) cancerous cells in the recipient. The recipient may be the same subject from which the cells was obtained (autologous cell therapy) or may be from another subject of the same species.

The immune cells, preferentially T cells or NK cells engineered to express the CAR as disclosed herein may be administered either alone, or as a pharmaceutical composition in combination with diluents and/or with other components such as IL-2 or other cytokines or cell populations. Briefly, pharmaceutical compositions of the present invention may comprise a cell population of genetically modified cells (a plurality of immune cells) as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions of the present disclosure, but not part of the invention, are formulated for intravenous administration. The administration of cell compositions to the subject may be carried out in any convenient manner known in the art.

Pharmaceutical compositions of the present disclosure, but not part of the invention, may be administered in a manner appropriate to the disease to be treated. Appropriate dosages may be determined by clinical trials. But the quantity and frequency of administration will also be determined and influenced by such factors as the condition of the patient, and the type and severity of the patient's disease.

A pharmaceutical composition comprising the immune cells, preferentially T cells or NK cells as disclosed herein may be administered at a dosage of 10⁴ to 10⁹ cells/kg body weight, preferably 10⁵ to 10⁶ cells/kg body weight. The cell compositions may also be administered several times at these dosages. The compositions of cells may be injected e.g. directly into a tumor, lymph node, or site of infection.

The genetically engineered immune cells may be activated and expanded to therapeutic effective amounts using methods known in the art.

The immune cells of the disclosure may be used in combination with e.g. chemotherapy, radiation, immunosuppressive agents, antibodies or antibody therapies.

"Universal" CAR systems (or adapter CAR systems) that indirectly bind to target cells via soluble factors are described in the art. E.g. in WO2012082841A2, WO2013044225A1 and WO2016030414A1 tagged antibodies and tag-specific CAR are disclosed, wherein the tag may be either artificial (such as FITC) and potentially immunogenic or an endogenous molecule which may compete with the natural counterparts to the CAR binding.

Therefore, in another aspect the present disclosure, but not part of the invention, provides a composition comprising
i) an immune cell comprising a CAR comprising
   a) an antigen binding domain specific for a tag of a tagged polypeptide
   b) a spacer
   c) a transmembrane domain
   d) an intracellular signaling domain,
   wherein said spacer comprises at least one C2-set Ig-like domain,
ii) said tagged polypeptide, wherein said polypeptide binds specifically to an antigen.

Said composition as disclosed herein, wherein said tagged polypeptide may be an antibody or antigen binding fragment thereof.

Said composition as disclosed herein, wherein said tag of said tagged polypeptide may be a hapten.

Said tagged polypeptide may comprise SEQ ID NO:5 or SEQ ID NO: 10.

Said tag may be selected from the group consisting of dextran, biotin, fluorescein isothiocyanate (FITC), phycoerythrin (PE), peptides such as c-Myc-tag, Strep-Tag, Flag-Tag, Polyhistidine-tag or proteins such as streptavidin. Preferentially, the tag may be biotin or a derivate thereof.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs. As used herein the term "comprising" or "comprises" is used in reference to compositions, methods, and respective component(s) thereof, that are essential to the method or composition, yet open to the inclusion of unspecified elements, whether essential or not.

In general, a CAR may comprise an extracellular domain (extracellular part) comprising the antigen binding domain, a transmembrane domain and a cytoplasmic signaling domain (intracellular signaling domain). The antigen binding domain may be linked to the transmembrane domain by a spacer. The extracellular domain may also comprise a signal peptide. In some embodiments of the invention the antigen binding domain of a CAR binds a tag or hapten that is coupled to a polypeptide ("haptenylated" or "tagged" polypeptide), wherein the polypeptide may bind to a disease-associated antigen such as a tumor associated antigen (TAA) that may be expressed on the surface of a cancer cell.

Such a CAR may be referred to as "anti-tag" CAR or "adapterCAR" or "universal CAR" as disclosed e.g. in US9233125B2.

The haptens or tags may be coupled directly or indirectly to a polypeptide (the tagged polypeptide), wherein the polypeptide may bind to said disease associated antigen expressed on the (cell) surface of a target. The tag may be e.g. dextran or a hapten such as biotin or fluorescein isothiocyanate (FITC) or phycoerythrin (PE), but the tag may also be a peptide sequence e.g. chemically or recombinantly coupled to the polypeptide part of the tagged polypeptide. The tag may also be streptavidin. The tag portion of the tagged polypeptide is only constrained by being a molecular that can be recognized and specifically bound by the antigen binding domain specific for the tag of the CAR. For example, when the tag is FITC (Fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA). The CARs of the invention may comprise one or more antigen binding domains (e.g. a tandem CAR). Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S)₃-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain and the transmembrane domain. The CARs of the invention comprise an extracellular spacer as disclosed herein, i.e. it consists of 1, 2 or 3 C2-set Ig-like domain, wherein said C2-set Ig-like domain(s) is/are selected from the C2-set Ig-like domain(s) of Siglec-4, wherein said spacer does not comprise a C1-set Ig-like domain. This is in contrast to regularly used spacer of the art, where the spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

A C2-set Ig-like domain is Ig-like domain that is characterized by seven antiparallel beta-strands, presented in two beta-sheets of three (ABE) and four (CC'FG) strands (FIG 10).

A C1-set Ig-like domain is Ig-like domain that is characterized by eight antiparallel beta-strands, presented in two beta-sheets of four (ABED) and four (CC'FG) strands (FIG 10).

The immunoglobulin superfamily (IgSF) is a large group of soluble and cell surface proteins that share a common structural feature, that was originally described in antibodies: the Ig fold. The overall shape of the domain can be described as a flattened cylinder or a -barrel. The basic folding motif consists of two layers of β-sheets with antiparallel β-strands that are stabilized by hydrophobic interactions and a central disulfide bond between two opposing strands. A β-sheet is commonly composed of 3-5 antiparallel β-strands, and 5-10 amino acids usually make up one β-strand. The strands are labeled alphabetically, starting from the N-terminus. Surface exposed amino acid loops connect the β-strands. The disulfide bridge is formed by two conserved cystein residues that are separated by a stretch of 55-75 amino acids from each other in the primary sequence.

Ig-like domains are protein domains that are similar to the amino acid sequence and structure of Igs. Ig-like domains are categorized into four different topological subtypes that are called "sets": the V-set (antibody variable-domain like), the C1-set (antibody constant-domain 1 like), the C2-set (antibody constant-domain 2 like), and the I-set domain (antibody intermediate-domain like). Structurally, sets differ in the number and size of the β-strands as well as the size and conformation of the loops. A V-set domain is generally larger than C-set domains and possesses a total of 9 β-strands. The β-strands designated as A, B, E, and D are on one sheet and G, F, C, C', and C" on the other β-sheet. The two additional strands that are missing in C1-set domains are located between strand C and D and therefore are called C' and C". Another hallmark of a V-set domain is the A-A' kink in the N-terminal A strand. In many cases a cis-Prolin resides at the kink. Cysteines of V-set folds are generally spaced apart at 65-75 amino acids.

C1- and C2-set folds have a total of seven β-strands and in general, their cysteines are spaced 55-60 amino acids apart in the linear sequence. In case of C1-set domains, the first β-sheet is composed of four β-strands designated as A, B, D, and E, while the second is composed of three β-strands designated as C, F, and G.

C2-set domains combine features of the V- and the C1-set domains: the fold corresponds to the C1-set domain, but amino acid sequences of the β-strands near the C-terminal conserved cystein residue are similar to the V-set domain. Thus, a folding structure arises that forms one β-sheet composed of the β-strands E, B, A and a second β-sheet composed of the strands G, F, C, C'. This architecture generally shows a tighter association between the E and the F strands.

Members of the I-set domain are more closely related to the V-set domains and the fold can be described as a V-set domain which is truncated on one side resulting in a missing C" strand (Natarajan 2015, Bork 1994, Huang 1997).

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The splitting key signaling and antigen recognition modules enable for a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are that those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, PD-1, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some embodiments, the endodomain may contain a primary cytoplasmic signaling domains or a co-stimulatory region, but not both. **In** these embodiments, an immune effector cell containing the disclosed CAR is only activated if another CAR containing the missing domain also binds its respective antigen.

In a disclosure, but not part of the invention, the CAR may be a "SUPRA" (split, universal, and programmable) CAR, where a "zipCAR" domain may link an intra-cellular costimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

If the CAR is an inhibitory CAR (referred to herein normally as "iCAR"), then said CAR may have the same extracellular and/or transmembrane domains as the activating CAR but differs from the activating CAR with regard to the endodmain.

The at least one endodomain of the inhibitory CAR may be a cytoplasmic signaling domain comprising at least one signal transduction element that inhibits an immune cell or comprising at least one element that induces apoptosis.

Inhibitory endodomains of an iCAR are well-known in the art and have been described e.g. in WO2015075469A1, WO2015075470A1, WO2015142314A1, WO2016055551A1, WO2016097231A1, WO2016193696A1, WO2017058753A1, WO2017068361A1, WO2018061012A1, and WO2019162695A1.

Said at least one signal transduction element that inhibits or may be capable of inhibiting an (effector) immune cell of said iCAR may be a signal transduction element of an immune checkpoint protein.

Said inhibitory signal transduction element may be selected from the groups consisting of:
- the immunoglobulin superfamily (IgSF) and tumour necrosis factor receptor superfamily (TNFRSF) including immune checkpoint proteins CD22, CD31, CD33, CD47, CD85A (LIR3), CD85C (LIR8), CD85D (LIR2), CD87J (LIR1), CD85K (LIR5), CD89 (B71), CD94 (KLRD1), CD152 (CTLA4), CD158A (KIR2DL1), CD158B1 (KIR2DL2), CD158B2 (KIR2DL3), CD158D (KIR2DL4), CD158E1 (KIR3DL1), CD158F (KIR2DL5A) CD158K (KIR3DL2), CD158Z (KIR3DL3), CD159a, CD159c, CD160, CD223 (LAG3), CD244 (SLAMF4), CD272 (BTLA), CD274 (PDL1), CD279 (PD1), CD328 (Siglec7), CD329 (Siglec9), CD352 (SLAMF6), CEACAM1, CEACAM2, FcgammaR, G6b-B, KIR2DL5B, KLRG1, LAIR1, PD1H (Vista), PIR-B, Siglec2, Siglec3, Siglec5, Siglec6, Siglec8, Siglec10, Siglec11, Siglec12, TIGIT, TIM2, TIM3, and TLT-1
- protein tyrosine phosphatases ACP1, CDC14A, CDC14B, CDC14C, CDC25A, CDC25B, CDC25C, CDKN3, DNAJC6, DUPD1, DUSP1, DUSP10, DUSP11, DUSP12, DUSP13, DUSP14, DUSP15, DUSP16, DUSP18, DUSP19, DUSP2, DUSP21, DUSP22, DUSP23, DUSP26, DUSP27, DUSP28, DUSP3, DUSP4, DUSP5, DUSP6, DUSP7, DUSP8, DUSP9, EPM2A, FIG4, GAK, INPP5A, INPP5B, INPP5D, INPP5E, INPP5F, INPP5J, INPP5K, INPPL1, MTM1, MTMR1, MTMR10, MTMR11, MTMR12, MTMR14, MTMR2, MTMR3, MTMR4, MTMR6, MTMR7, MTMR8, MTMR9, OCRL, PALD1, PIP4P1, PIP4P2, PTEN, PTP4A1, PTP4A2, PTP4A3, PTPDC1, PTPMT1, PTPN1, PTPN11, PTPN12, PTPN13, PTPN14, PTPN18, PTPN2, PTPN20, PTPN21, PTPN22, PTPN23, PTPN3, PTPN4, PTPN5, PTPN6, PTPN7, PTPN9, PTPRA, PTPRB, PTPRC, PTPRD, PTPRE, PTPRF, PTPRG, PTPRH, PTPRJ, PTPRK, PTPRM, PTPRN, PTPRN2, PTPRO, PTPRQ, PTPRR, PTPRS, PTPRT, PTPRU, PTPRZ1, RNGTT, SACM1L, SBF1, SBF2, SSH1, SSH2, SSH3, STYX, STYXL1, SYNJ1, SYNJ2, TNS1, TNS2, TNS3, TNS4, TPTE, and TPTE2.

Said at least one signal transduction element that inhibits an immune cell of said iCAR may be also selected from STimulator of INterteron Genes (STING); immunoreceptor tyrosine-based inhibitory motif (ITIM) containing proteins, immunoreceptor tyrosine-based switch motif (ITSM) containing proteins, T cell immunoglobulin and IITM domain (TIGIT), and adenosine receptor (e.g. A2aR).

Said at least one signal transduction element that inhibits an immune cell of said iCAR may be also a tyrosine phosphatase domain from a Src homolog (SH2) domain-containing protein tyrosine phosphatase which is recruited by a phosphorylated Immunoreceptor Tyrosine-based Activation motif (ITIM).

Said at least one signal transduction element that inhibits an immune cell of said iCAR may be also
(i) a truncated protein which comprises an SH2 domain from a protein which binds a phosphorylated immunoreceptor tyrosine-based activation motif (ITAM), but lacks a kinase domain; or
(ii) a truncated protein which comprises an SH2 domain from a protein which binds a phosphorylated immunoreceptor tyrosine-based inhibition motif (ITIM) but lacks a phosphatase domain; or
(iii) a fusion protein which comprises (a) an SH2 domain from a protein which binds a phosphorylated immunoreceptor tyrosine-based activation motif (ITAM) or from a protein which binds a phosphorylated immunoreceptor tyrosine-based inhibition motif (ITIM); and (ii) a heterologous domain. Said heterologous domain may be a phosphatase domain or a kinase domain.

Said at least one element that induces apoptosis may be e.g. a Tumor-necrosis-factor related apoptosis inducing ligand (TRAIL) receptor or a CD200 receptor as described e.g. in detail in WO20160972331A1.

The CARs of the present invention may be designed to comprise any portion or part of the above-mentioned domains as described herein in addition to the spacer as disclosed herein in any order and/or combination resulting in a functional CAR, i.e. a CAR that mediated an immune effector response of the immune effector cell that expresses the CAR as disclosed herein or that as an inhibitory function (iCAR) as disclosed herein.

The term "tagged polypeptide" as used herein refers to a polypeptide that has bound thereto directly or indirectly at least one additional component, i.e. the tag. The tagged polypeptide as used herein is able to bind an antigen expressed on a target cell. The polypeptide may be an antibody or antigen binding fragment thereof that binds to an antigen expressed on the surface of a target cell such as a tumor associated antigen on a cancer cell. The polypeptide of the tagged polypeptide alternatively may a cytokine or a growth factor or another soluble polypeptide that is capable of binding to an antigen of a target cell.

The term "adapter" or "adapter molecule" as used herein refers to a tagged polypeptide that can bind to an antigen of a target cell, e.g. antibody or antigen binding fragment thereof, and has bound thereto directly or indirectly at least one additional component, i.e. the tag. The adapter or adapter molecule may by a tagged antibody or antigen binding fragment thereof, a cytokine or a growth factor or another soluble polypeptide that is capable of binding to an antigen of a target cell.

The tag may be e.g. a hapten or dextran and the hapten or dextran may be bound by the antigen binding domain of the polypeptide comprising an antigen binding domain specific for the tag.

Haptens such as e.g. FITC, biotin, PE, streptavidin or dextran are small molecules that elicit an immune response only when attached to a large carrier such as a protein; the carrier may be one that also does not elicit an immune response by itself. Once the body has generated antibodies to a hapten-carrier adduct, the small-molecule hapten may also be able to bind to the antibody, but it will usually not initiate an immune response; usually only the hapten-carrier adduct can do this.

But the tag may also be a peptide sequence e.g. chemically or recombinantly coupled to the polypeptide part of the tagged polypeptide. The peptide may be selected from the group consisting of c-Myc-tag, Strep-Tag, Flag-Tag, and Polyhistidine-tag. The tag may also be streptavidin. The tag portion of the tagged polypeptide is only constrained by being a molecular that can be recognized and specifically bound by the antigen binding domain specific for the tag of the CAR. For example, when the tag is FITC (Fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies, diabodies, dsFv, Fab', diabodies, single-chain antibody molecules, and multispecific antibodies formed from antibody fragments.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain as specific. An antigen-binding domain that specifically binds to an antigen may bind also to different allelic forms of the antigen (allelic variants, splice variants, isoforms etc.). This cross reactivity is not contrary to the definition of that antigen-binding domain as specific.

T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface. There are several subsets of T cells, each with a distinct function.

T helper cells (TH cells) assist other white blood cells in immunologic processes, including maturation of B cells into plasma cells and memory B cells, and activation of cytotoxic T cells and macrophages. These cells are also known as CD4+ T cells because they express the CD4 glycoprotein on their surface. Helper T cells become activated when they are presented with peptide antigens by MHC class II molecules, which are expressed on the surface of antigen-presenting cells (APCs). Once activated, they divide rapidly and secrete small proteins called cytokines that regulate or assist in the active immune response. These cells can differentiate into one of several subtypes, including TH1, TH2, TH3, TH17, Th9, or TFH, which secrete different cytokines to facilitate a different type of immune response. Signaling from the APC directs T cells into particular subtypes.

Cytotoxic T cells (TC cells, or CTLs) destroy virally infected cells and tumor cells, and are also implicated in transplant rejection. These cells are also known as CD8+ T cells since they express the CD8 glycoprotein at their surface. These cells recognize their targets by binding to antigen associated with MHC class I molecules, which are present on the surface of all nucleated cells.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - Foxp3+ Treg cells and Foxp3-Treg cells.

Natural killer T cells (NKT cells - not to be confused with natural killer cells of the innate immune system) bridge the adaptive immune system with the innate immune system. Unlike conventional T cells that recognize peptide antigens presented by major histocompatibility complex (MHC) molecules, NKT cells recognize glycolipid antigen presented by a molecule called CD1d. Once activated, these cells can perform functions ascribed to both Th and Tc cells (i.e., cytokine production and release of cytolytic/cell killing molecules).

The term "natural killer cells (NK cells)" are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter into the circulation. NK cells differ from natural killer T cells (NKTs) phenotypically, by origin and by respective effector functions; often, NKT cell activity promotes NK cell activity by secreting IFNγ. In contrast to NKT cells, NK cells do not express T-cell antigen receptors (TCR) or pan T marker CD3 or surface immunoglobulins (Ig) B cell receptors, but they usually express the surface markers CD16 (FcγRIII) and CD56 in humans, NK1.1 or NK1.2 in C57BL/6 mice. Up to 80% of human NK cells also express CD8. Continuously growing NK cell lines can be established from cancer patients and common NK cell lines are for instance NK-92, NKL and YTS.

The terms "immune cell" or "immune effector cell" may be used interchangeably and refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. "Effector function" means a specialized function of a cell, e.g. in a T cell an effector function may be cytolytic activity or helper activity including the secretion of cytokines.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "epitope" means the part of an antigen, e.g. a soluble antigen, that may be recognized and specifically bound by antibodies or antigen bindings fragments thereof (antigen binding domains).

The tumor associated antigen (TAA) as used herein refers to an antigenic substance produced in tumor cells. Tumor associated antigens are useful tumor or cancer markers in identifying tumor/cancer cells with diagnostic tests and are potential candidates for use in cancer therapy. Preferentially, the TAA may be expressed on the cell surface of the tumor/cancer cell, so that it may be recognized by the antigen binding receptor as disclosed herein.

The term "target cell" as used herein refers to cell which expresses an antigen on its cell surface that should be recognized (bound) by the antigen binding domain of the CAR as disclosed herein or by the antigen binding domain of the tag of the tagged polypeptide as disclosed herein. Said target cell may be e.g. a cancerous cell or a cell associated with an autoimmune disease or a cell associated with an infectious disease.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. T cells that have a natural or genetically engineered reactivity to a patient's cancer are generated *in-vitro* and then transferred back into the cancer patient. Then the immunotherapy is referred to as "CAR immunotherapy" or in case of use of T cells only as "CAR T cell therapy" or "CAR T cell immunotherapy".

The term "treatment" as used herein means to reduce the frequency or severity of at least one sign or symptom of a disease.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the individual is a human. The subject may be a subject suffering from a disease such as cancer. The term "expression" as used herein is defined as the transcription and/or translation of a particular nucleotide sequence driven by its promoter in a cell.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface.

The term "cancer" is known medically as a malignant neoplasm. Cancer is a broad group of diseases involving unregulated cell growth and includes all kinds of leukemia. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

### Examples

The following examples are intended for a more detailed explanation of the invention but without restricting the invention to these examples.

### Example 1: Construction and expression of C2-set Ig-like domain spacer CARs in HEK293 and primary human T cells

Commercial gene synthesis in combination with an optimization algorithm for codon usage in humans (ATUM) was used to construct C2-set Ig-like domain spacer and control CAR genes targeting the CD20 antigen. The CD20-specific CARs comprised as a binding domain SEQ ID NO:5, which binds a well-characterized membrane-proximal epitope. All antigen binding domains contained a (G4S)3-linker between the VL and the VH regions. To facilitate receptor trafficking to the plasma membrane, a human CD8α or CD8β leader signaling peptide was added N-terminally to the respective scFv sequence. The spacer region downstream of the scFv for the state-of-the-art control CD20 CAR encompassed the CD8α hinge (45 amino acids). Spacers bearing C2-set Ig-like domains were derived from the Siglec family and designed based on the protein sequences extracted from UniProt and the plasma membrane-proximal domains were incorporated into the CAR architecture. Thus, the Siglec-3 spacer comprised the amino acids 145-259 of the parent protein with a C169S mutation to abrogate unspecific disulfide-bond formation (SEQ ID NO:12). The Siglec-4 spacer contained the amino acids 238-519 (SEQ ID NO:1), the Siglec-7.1 spacer the amino acids 150-353 (SEQ ID NO:2), the Siglec-7.2 spacer the amino acids 234-353 (SEQ ID NO:3), and the Siglec-8 spacer the amino acids 241-363 (SEQ ID NO:4) of the respective parent protein. All spacers were linked to the transmembrane domain of human CD8α, the intracellular domain of 4-1BB, and the CD3ζ signaling domain as derived from UniProt (SEQ ID NO:6-9; SEQ ID NO:13). The CAR genes were fused to a Furin-P2A sequence to include co-expression of the truncated low affinity nerve growth factor receptor (ΔLNGFR). Transgene expression was promoted by the PGK promoter located upstream of the CAR gene.

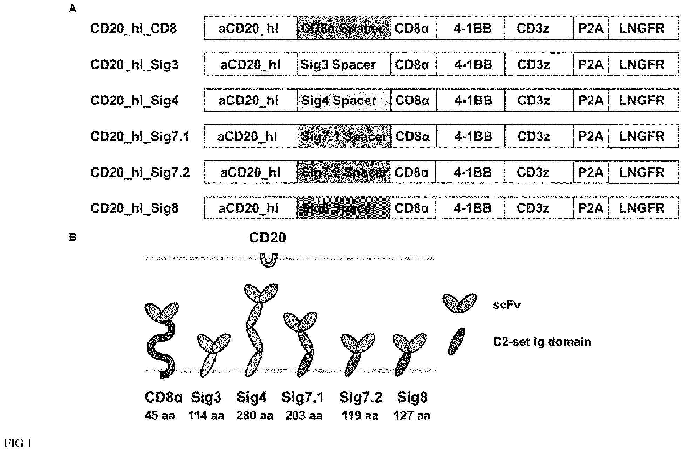

To confirm correct translation and surface expression of the constructs, bicistronic lentiviral expression vectors were generated (FIG. 1). After transfection of the DNA constructs into HEK293T cells, detection of the reporter protein ΔLNGFR confirmed successful transcription and translation of the CAR cassette, while direct staining of the CAR with a CD20 CAR detection reagent (PE) visualized surface expression of the CAR constructs. All constructs showed both ΔLNGFR and CAR expression in >80 % of HEK293T cells (FIG. 2A). To analyse CAR construct expression in primary T cells, peripheral blood mononuclear cells (PBMCs) were isolated from buffy coats by density gradient centrifugation. T cells were purified from PBMCs applying the Pan T Cell Isolation Kit, human (Miltenyi Biotec) and activated in TexMACS^{™} Medium (Miltenyi Biotec) supplemented with T Cell TransAct^{™}, human (Miltenyi Biotec) and 100 IU/ml of recombinant Human IL-2 IS, research grade (Miltenyi Biotec). T cells were transduced 24 h after activation using VSV-G pseudotyped lentiviral particles. 3 days post activation, T Cell TransAct^{™}, human and excess viral vector were removed and T cells were cultured in TexMACS^{™} Medium only supplemented with IL-2. T cells were expanded for 12 days and used directly for in vitro assays. The ΔLNGFR reporter protein was expressed in all cases demonstrating effective lentiviral transduction of the T cells and translation of the expression cassette (FIG. 2B). While three CAR constructs showed CAR expression levels comparable to the CD8α spacer CAR control (CD20_hl_Sig4 CAR, CD20_hl_Sig7.2 CAR, CD20_hl_Sig8 CAR), no CD20_hl_Sig7.1 CAR expression was detectable and the CD20_hl_Sig3 CAR was expressed on only 5 % of the T cells.

### Example 2: C2-set Ig-like domain spacer CARs targeting CD20 are functional in primary human T cells

2x10⁵ T cells, each expressing different Siglec-based spacer CAR constructs or a CD8α hinge spacer CAR control, were incubated with 2x10⁵ CD20+ Raji cells in 200 µl TexMACS^{™} Medium at 37°C. In addition, the medium was supplemented with 20 µl of a CD107a specific antibody. After 1 h of incubation the protein transport inhibitors Monensin and Brefeldin A (BD Biosciences) were added as recommended for 4 h. After this incubation period, cells were washed and first surface stained with LNGFR specific antibodies to label transduced T cells and subsequently intracellularly stained for TNF-α using the Inside Stain Kit and a TNF-α specific antibody (all Miltenyi Biotec). Cells were then measured by flow cytometry. For analysis of the T cell activation/exhaustion markers TIM3, LAG3 and PD1, 1x10⁵ CAR T cells were inoculated with 2x10⁵ CD20+ Raji cells in 200 µl TexMACS^{™} Medium at 37°C for 24 h. Subsequently T cells were stained and analysed by flow cytometry. Only CAR T cells co-cultured with CD20+ target cells showed significant degranulation (FIG. 2C). Strongest degranulation could be observed for the CD8α and Siglec-4 spacer variants with around 35 % of CD107α positive cells. The Siglec-7.2 spacer CAR produced an intermediate amount of CD107α at 20 % positive cells and the Siglec-8 variant showed a lower 10% degranulation that was still significantly different to the negative control cells. Similar to the degranulation analysis, the proportion of ΔLNGFR+/TNF-α+ cells was also highest in CD8α spacer CAR T cells (32%, FIG. 2 D) but markedly fewer CD20_hl_Sig4 CAR T cells expressed TNF-α (18% positive cells), followed by Siglec-7.2 and Siglec-8 spacer CARs. Again, no unspecific activation could be observed in the controls. We also assessed the activation state of the modified T cells by analysing TIM3, LAG3 and PD1 surface expression. CD20+ Raji cells were co-cultured with CAR T cells for 24 h at an E:T ratio of 1:2. The CD8α and Siglec-4 spacer CAR modified T cells contained the largest fraction of TIM3/LAG3/PD1 triple positive cells (FIG. 2E).

### Example 3: C2-set Ig-like domain spacer CARs targeting TSPAN8 are functional in primary human T cells

The Siglec-based spacers were tested in the context of a solid tumour model to target novel pancreatic ductal adenocarcinoma (PDAC) target antigens CD66c and TSPAN8. TSPAN8 has two extracellular loops extending from the membrane that span 24 and 96 amino acids respectively, the larger having two interconnecting disulfide bonds. Thus, the whole protein is very membrane proximal. On the other hand, CD66c is a glycophosphatidylinositol anchored protein and consists of two C2-set domains and one V-set domain. In consequence it extends further into the extracellular space compared to TSPAN8. In addition, the epitope of the aCD66c scFv is localized on the outer N terminal V-set domain. In summary, TSPAN8 can be considered a membrane proximal target, while CD66c is a membrane distal target.

Siglec-4 spacer CARs targeting TSPAN8 (SEQ ID NO:11) or CD66c were generated by replacing the scFv from the CD20_hl_Sig4 CAR (SEQ ID NO:6) through the scFv derived from the TSPAN8-reactive monoclonal antibody REA443 (SEQ ID NO:10) or the CD66c-reactive monoclonal antibody REA414 (Miltenyi Biotec). Additionally, we incorporated in our experiments CD66c and TSPAN8 specific CD8 α spacer CARs and a TSPAN8 specific IgG4

CH2-CH3 spacer CAR of 228 amino acids (FIG. 3). In the case of the IgG4 CH2 domain, the APEFLG sequence was replaced by APPVA from IgG2 and an N279Q mutation was introduced to remove glycosylation at this site (Hudecek et al., 2015).

GFP+/Luc+ AsPC1 target cells were inoculated in 96-well plates at 2.5x10⁴ cells per well in TexMACS^{™} Medium. CAR T cells or untransduced Mock T cells were added with at an E:T ratio of 2:1. The amount of T cells in the Mock control was adjusted to the number of total T cells in the CAR group with the highest total cell count. Cytotoxicity was measurement as the decrease of green surface area as assessed by the IncuCyte^{®} S3 Live-Cell Analysis System (Sartorius). Measured values were normalized to the start of the experiment. After 24 h a supernatant sample was taken for cytokine measurements using the MACSPlex Cytokine 12 Kit. At the end of the experiment expression of LAG3, PD1 and 4-1BB were measured using a MACSQuant Analyzer 8 (Miltenyi Biotec). Specific endpoint killing was calculated from the green surface area values with the following formula: $specific killing \left[%\right] = 100 - \left(100∗\frac{green area Mock}{green area CAR}\right)$

Both, the CD66c_lh_Siglec-4 CAR T cells, as well as the untransduced control T cells showed no specific killing of target cells, while the CD66c_lh_CD8 CAR showed a specific endpoint killing of 42 %, (FIG. 4). On the other hand, when targeting the membrane proximal TSPAN8, the Siglec-4 spacer CAR T cells showed a similar killing to that of the TSPAN8_hl_CD8α CAR T cells approaching 60 % endpoint killing. In contrast, CAR T cells modified with a TSPAN8 CAR with the alternative long IgG4 CH2-CH3 spacer exhibited only 40 % killing at the end of the experiment, showing the weakest cytotoxicity of all tested TSPAN8 CAR T cells. The CD66c_lh_Sig4 CAR T cells, which showed no cytotoxicity, also expressed no activation markers (FIG. 5). The strongest upregulation of activation markers 4-1BB, LAG3 and PD-1 was observed in TSPAN8_hl_CD8α CAR T cells. Interestingly and unexpectedly, the TSPAN8 specific Siglec-4 CAR T cells displayed a lower expression of activation markers, even though the cytotoxicity equalled that of the CD8α spacer CAR T cells. This difference between the CD8α and the Siglec-4 spacer CAR T cells was even more striking at the cytokine level (FIG. 6). The TSPAN8_hl_CD8 CAR T cells released markedly higher levels of cytokines than the other CAR T cells. The TSPAN8_hl_Sig4 CAR T cells, despite displaying similar killing kinetics to the TSPAN8 CD8α CAR T cells, secreted over 10-fold lower levels of cytokines after antigen-specific stimulation, which was very surprising, given the same observed cytotoxicity as the TSPAN8 CD8α CAR T cells.

### Example 4: C2-set Ig-like domain spacer CARs targeting TSPAN8 are highly functional in an in vivo solid tumor model

We investigated the functionality of three TSPAN8 specific CAR constructs TSPAN8_hl_Sig4 CAR, TSPAN8_hl_IgG4 CAR and TSPAN8 CD8α CAR) in vivo in a pre-clinical PDAC tumor model. For AsPC1 GFP+/Luc+ cell line derived tumors 1x10⁶ cells were injected subcutaneously in the right flank of NOD SCID gamma (NSG; NOD.Cg-PrkdescidIl2rgtm1Wjl/SzJ) mice (Jackson Laboratory, provided by Charles River). When tumors reached a size of 25 mm², 5x10⁶ CAR T cells were injected into the tail vein. The amount of injected untransduced Mock T cells was adjusted to the number of total T cells in the CAR group with the highest total cell count (FIG. 7).

Therapeutic response was measured longitudinally using the IVIS Lumina in vivo imaging system (PerkinElmer) after intraperitoneal injection of 100 µL (30 mg/mL) D-Luciferin (Potassium Salt, LUCK-2G, GoldBio) and additionally by manual caliper measurement (FIG. 8). All measures to secure the well-being of mice were executed following the relevant animal use guidelines and ethical regulations. Upon reaching the endpoint (weight loss of >19%, paralysis, stress score of >20 or end-point of the experiment, Day 20 for the lymphoma model and Day 29 for the pancreatic model), animals were euthanized according to guidelines and post-mortem analysis was performed in order to determine tumor burden, persistence and killing of the different CAR T cell constructs. Therefore, spleen was dissociated using the gentleMACS^{™} Octo Dissociator with Heaters according to the manufacturers protocol (Miltenyi Biotec). The cell suspensions were filtered through a 70 µm pore size MACS SmartStrainer (Miltenyi Biotec) and following red blood cell lysis on spleen single cell suspensions using Red Blood Cell Lysis Solution (Miltenyi Biotec), samples were stained and analysis was conducted on a MACSQuant Analyzer 8.

Untransduced (Mock) T cells did not display a therapeutic benefit over the untreated group (FIG. 8). All mice from these two groups had to be sacrificed before the end of the experiment as tumor ulcerations began to become established. The therapeutic effect for the CD8α and Siglec-4 CARs became apparent in BLI measurements from day 6 onwards. The tumor burden within the groups treated with the CD8α and Siglec-4 spacer CARs decreased in a comparable manner and reached baseline by the end of the experiment 29 days after T cell injection. At the same time, tumor growth was controlled by the IgG4 CH2-CH3 spacer group, but there was no tumor clearance as seen with the other groups. Persistence of CAR T cells could be demonstrated in the spleens of all CAR T cell treated groups with the highest amounts found in the CD8α spacer CAR and Siglec-4 spacer CAR groups (FIG. 9). A markedly lower amount of CAR T cells could be recovered from the IgG4 spacer CAR group. Interestingly, when the phenotype of the human T cells was examined the proportion of TCM (central memory T cells) was twice as high in CD4 and CD8 CAR T cells of the Siglec-4 spacer CAR group as compared to the CD8α spacer CAR T cells (FIG. 9). This is of particular interest as the TCM phenotype is associated with better overall remission and decreased likelihood of relapse in a clinical context.

### References

Gross, G., Waks, T., and Eshhar, Z. (1989). Expression of immunoglobulin-T-cell receptor chimeric molecules as functional receptors with antibody-type specificity. Proceedings of the National Academy of Sciences of the United States of America 86(24), 10024-10028. doi: 10.1073/pnas.86.24.10024.
Hudecek, M., Sommermeyer, D., Kosasih, P.L., Silva-Benedict, A., Liu, L., Rader, C., et al. (2015). The nonsignaling extracellular spacer domain of chimeric antigen receptors is decisive for in vivo antitumor activity. Cancer immunology research 3(2), 125-135. doi: 10.1158/2326-6066.CIR-14-0127.
Patel, S.D., Moskalenko, M., Smith, D., Maske, B., Finer, M.H., and McArthur, J.G. (1999). Impact of chimeric immune receptor extracellular protein domains on T cell function. Gene therapy 6(3), 412-419. doi: 10.1038/sj.gt.3300831.
Watanabe, N., Bajgain, P., Sukumaran, S., Ansari, S., Heslop, H.E., Rooney, C.M., et al. (2016). Fine-tuning the CAR spacer improves T-cell potency. Oncoimmunology 5(12), e1253656-e1253656. doi: 10.1080/2162402X.2016.1253656.
Krummel MF and Cahalan MD (2010) The Immunological Synapse: a Dynamic Platform for Local Signaling. J Clin Immunol 30:364-72
Garcia KC, Degano M, Stanfield RL, Brunmark A, Jackson MR, Peterson PA, Teyton L, Wilson IA. An αβ T cell receptor structure at 2.5Å resolution and its orientation in the TCR-MHC complex. Science. 1996;274:209-219
Hudecek M, Lupo-Stanghellini MT, Kosasih PL, Sommermeyer D, Jensen MC, Rader C, Riddell SR. (2013) Receptor Affinity and Extracellular Domain Modifications Affect Tumor Recognition by ROR1-specific Chimeric Antigen Receptor T Cells. Clin Cancer Res 19:3153-64.
Natarajan, K.; Mage, M.G.; and Margulies, D.H. (April 2015) Immunoglobulin Superfamily. In: eLS. John Wiley & Sons, Ltd: Chichester.
Bork, P.; Holm, L.; Sander C. (Sept 1994) The Immunoglobulin Fold. Structural Classification, Sequence Patterns and Common Core
Huang, Z.; Li, S.; Korngold, R. (1997) Imunoglobulin Superfamily Proteins: Structure, Mechanisms, and Drug Discovery

## Claims

1. A CAR comprising
a) an antigen binding domain specific for an antigen
b) a spacer
c) a transmembrane domain
d) an intracellular signaling domain,
wherein said spacer consists of 1, 2 or 3 C2-set Ig-like domain(s), wherein said C2-set Ig-like domain(s) is/are selected from the C2-set Ig-like domain(s) of Siglec-4.

2. The CAR according to claim 1, wherein said antigen is an antigen expressed on the surface of a target cell such as a cancer cell.

3. The CAR according to claim 1 or 2, wherein said antigen binding domain and said spacer are from different proteins.

4. The CAR according to claim 1 to 3, wherein said spacer comprises SEQ ID NO:1.

5. The CAR according to any one of claims 1 to 4, wherein said antigen binding domain is a scFv.

6. The CAR according to any one of claims 1 to 5, wherein said intracellular signaling domain comprises a primary signaling domain and a co-stimulatory signaling domain.

7. The CAR according to any one of claims 1 to 6, wherein said antigen domain is specific for CD20.

8. The CAR according to claim 7, wherein said antigen binding domain is a scFv.

9. The CAR according to claim 8, wherein said scFV has the sequence of SEQ ID NO: 5.

10. The CAR according to any one of claims 1 to 6, wherein said antigen binding domain is specific for TSPAN8.

11. The CAR according to claim 10, wherein the antigen binding domain has the sequence of SEQ ID NO: 10.

12. The CAR according to claim 1 to 3, wherein said antigen binding domain is specific for an epitope on an antigen expressed on a cell membrane of a target cell, wherein said epitope is a relatively proximal epitope, and wherein said spacer comprises two or three C2-set Ig-like domains, and wherein said spacer does not comprise a C1-set Ig-like domain.

13. The CAR according to claim 12, wherein said spacer comprises SEQ ID NO:1.

14. The CAR according to claim 1 to 3, wherein said antigen binding domain is specific for an epitope on an antigen expressed on a cell membrane of a target cell, wherein said epitope is a relatively distal epitope, and wherein said spacer comprises one or two C2-set Ig-like domain(s), and wherein said spacer does not comprise a C1-set Ig-like domain.

## Patentansprüche

1. CAR, umfassend:
a) eine Antigenbindungsdomäne, die für ein Antigen spezifisch ist,
b) einen Spacer,
c) eine Transmembrandomäne,
d) eine intrazelluläre Signaldomäne,
wobei der Spacer aus 1, 2 oder 3 C2-Set-Ig-ähnlichen Domänen besteht, wobei die C2-Set-Igähnliche(n) Domäne(n) ausgewählt (ist) sind aus den C2-Set-Ig-ähnlichen Domänen von Siglec-4.

2. CAR nach Anspruch 1, wobei das Antigen ein Antigen ist, das auf der Oberfläche einer Zielzelle, wie beispielsweise einer Krebszelle, exprimiert wird.

3. CAR nach Anspruch 1 oder 2, wobei die Antigenbindungsdomäne und der Spacer aus unterschiedlichen Proteinen stammen.

4. CAR nach Anspruch 1 bis 3, wobei der Spacer SEQ ID NO:1 umfasst.

5. CAR nach einem der Ansprüche 1 bis 4, wobei die Antigenbindungsdomäne ein scFv ist.

6. CAR nach einem der Ansprüche 1 bis 5, wobei die intrazelluläre Signaldomäne eine primäre Signaldomäne und eine kostimulatorische Signaldomäne umfasst.

7. CAR nach einem der Ansprüche 1 bis 6, wobei die Antigendomäne spezifisch für CD20 ist.

8. CAR nach Anspruch 7, wobei die Antigenbindungsdomäne ein scFv ist.

9. CAR nach Anspruch 8, wobei der scFv die Sequenz von SEQ ID NO: 5 aufweist.

10. CAR nach einem der Ansprüche 1 bis 6, wobei die Antigenbindungsdomäne spezifisch für TSPAN8 ist.

11. CAR nach Anspruch 10, wobei die Antigenbindungsdomäne die Sequenz von SEQ ID NO: 10 aufweist.

12. CAR nach einem der Ansprüche 1 bis 3, wobei die Antigenbindungsdomäne spezifisch für ein Epitop auf einem Antigen ist, das auf einer Zellmembran einer Zielzelle exprimiert wird, wobei das Epitop ein relativ proximales Epitop ist und wobei der Spacer zwei oder drei C2-Set-Ig-ähnliche Domänen umfasst und wobei der Spacer keine C1-Set-Ig-ähnliche Domäne umfasst.

13. CAR nach Anspruch 12, wobei der Spacer SEQ ID NO: 1 umfasst.

14. CAR nach Anspruch 1 bis 3, wobei die Antigenbindungsdomäne spezifisch für ein Epitop auf einem Antigen ist, das auf einer Zellmembran einer Zielzelle exprimiert wird, wobei das Epitop ein relativ distales Epitop ist und wobei der Spacer eine oder zwei C2-Set-Ig-ähnliche Domänen umfasst und wobei der Spacer keine C1-Set-Ig-ähnliche Domäne umfasst.

## Revendications

1. CAR comprenant
a) un domaine de liaison à l'antigène spécifique d'un antigène
b) un espaceur
c) un domaine transmembranaire
d) un domaine de signalisation intracellulaire,
dans lequel ledit espaceur est constitué de 1, 2 ou 3 domaine(s) de type Ig de l'ensemble C2, dans lequel le(s)dit(s) domaine(s) de type Ig de l'ensemble C2 est/sont sélectionné(s) parmi le(s) domaine(s) de type Ig de l'ensemble C2 de Siglec-4.

2. CAR selon la revendication 1, dans lequel ledit antigène est un antigène exprimé à la surface d'une cellule cible telle qu'une cellule cancéreuse.

3. CAR selon la revendication 1 or 2, dans lequel ledit domaine de liaison à l'antigène et ledit espaceur sont de protéines différentes.

4. CAR selon les revendications 1 à 3, dans lequel ledit espaceur comprend la SEQ ID NO : 1.

5. CAR selon l'une quelconque des revendications 1 à 4, dans lequel ledit domaine de liaison à l'antigène est un scFv.

6. CAR selon l'une quelconque des revendications 1 à 5, dans lequel ledit domaine de signalisation intracellulaire comprend un domaine de signalisation primaire et un domaine de signalisation co-stimulatrice.

7. CAR selon l'une quelconque des revendications 1 à 6, dans lequel ledit domaine antigénique est spécifique de CD20.

8. CAR selon la revendication 7, dans lequel ledit domaine de liaison à l'antigène est un scFv.

9. CAR selon la revendication 8, dans lequel ledit scFV a la séquence de SEQ ID NO : 5.

10. CAR selon l'une quelconque des revendications 1 à 6, dans lequel ledit domaine de liaison à l'antigène est spécifique de TSPAN8.

11. CAR selon la revendication 10, dans lequel le domaine de liaison à l'antigène a la séquence de SEQ ID NO : 10.

12. CAR selon les revendications 1 à 3, dans lequel ledit domaine de liaison à l'antigène est spécifique d'un épitope sur un antigène exprimé sur une membrane cellulaire d'une cellule cible, dans lequel ledit épitope est un épitope relativement proximal, et dans lequel ledit espaceur comprend deux ou trois domaines de type Ig de l'ensemble C2, et dans lequel ledit espaceur ne comprend pas de domaine de type Ig de l'ensemble C1.

13. CAR selon la revendication 12, dans lequel ledit espaceur comprend la SEQ ID NO : 1.

14. CAR selon les revendications 1 à 3, dans lequel ledit domaine de liaison à l'antigène est spécifique d'un épitope sur un antigène exprimé sur une membrane cellulaire d'une cellule cible, dans lequel ledit épitope est un épitope relativement distal, et dans lequel ledit espaceur comprend un ou deux domaine(s) de type Ig de l'ensemble C2, et dans lequel ledit espaceur ne comprend pas de domaine de type Ig de l'ensemble C1.
